Europäisches Patentamt

European Patent Office

Office européen des brevets

(19)

(11) Publication number: **0 055 537**
**B1**

(12) **EUROPEAN PATENT SPECIFICATION**

(45) Date of publication of patent specification: **05.12.84**

(51) Int. Cl.³: **A 61 M 11/04**

(21) Application number: **81305785.8**

(22) Date of filing: **08.12.81**

(54) **Inhaler.**

(30) Priority: **12.12.80 JP 178213/80 u**
**12.12.80 JP 178214/80 u**
**29.01.81 JP 11335/81 u**

(43) Date of publication of application:
**07.07.82 Bulletin 82/27**

(45) Publication of the grant of the patent:
**05.12.84 Bulletin 84/49**

(84) Designated Contracting States:
**AT BE CH DE FR GB IT LI LU NL SE**

(56) References cited:
**FR-A-2 258 193**
**GB-A- 8 488**
**GB-A- 446 919**
**US-A-1 913 124**
**US-A-2 538 934**

(73) Proprietor: **Combi Co., Ltd.**
**No. 16-9, Uchikanda 3-chome Chiyoda-ku**
**Tokyo (JP)**

(72) Inventor: **Nakao, Shinroku**
**19-3, Kajiyama 1-chome Tsurumi-ku**
**Yokohama-shi Kanagawa (JP)**
Inventor: **Ishii, Yoshiyasu c/o Nido Industrial**
**Design**
**Working Company Limited, No. 21 Wakaba-cho**
**1-chome**
**Shinjuku-ku Toyko (JP)**

(74) Representative: **Carpenter, David et al**
**Marks & Clerk Alpha Tower Suffolk Street**
**Queensway**
**Birmingham B1 1TT (GB)**

EP 0 055 537 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to an inhaler.

A variety of inhalers designed to produce a stream of a mixture of chemical vapours together with steam for the purpose of maintaining one's health or producing a curative action on the throat, are known in the art. However, these conventional inhalers are disadvantageous in that since the vapour feeding sleeve of the inhaler, for guiding the stream of mixed vapours, is set at a fixed angle the user must position himself so as to correspond to the angle of the vapour feeding sleeve thereby to introduce the stream of vapours to his throat. Thus the user's posture is not natural when using the inhaler.

Prior published Patent Specifications GB—A—446919, GB—A—8488 and FR—A—2258193 show inhalers having tiltable sleeves but suffering from the disadvantage that they do not provide a storage position for the sleeve in which the sleeve is positioned within a removable cover so as to be protected thereby.

In producing a stream of mixed vapours with an ordinary inhaler, a chemical is sucked into a jet of steam produced by a vapour producing unit. The inhaler has a chemical container and a condensed water container in addition to the vapour producing unit. The conventional inhaler suffers from the further disadvantage that steam condensing on a chemical sucking nozzle inserted into the chemical container flows down into the chemical container, to dilute the chemical in the container.

The present invention is intended to minimise the above-described drawbacks of the conventional inhalers.

An inhaler according to the invention comprises a body including a vapour producing unit, a cover covering said vapour producing unit, and a vapour feeding sleeve for conducting vapour from the unit to a user's mouth in use, said sleeve being supported by said cover for tilting movement whereby the sleeve can be tilted at a desired angle for use, said cover is detachable from said body, and said sleeve can be rotated relative to said cover between a storage position wherein the sleeve is housed within the cover, and an operative position wherein the sleeve projects from the cover for use.

Thus the inhaler is so designed that its vapour feeding sleeve can be set out of the cover only when the inhaler is used, and can be set in the cover when it is not in use. Moreover, the vapour feeding sleeve can be tilted at a desired angle when using the inhaler.

Desirably said vapour producing body includes a vapour jetting nozzle and a chemical jetting nozzle, said vapour jetting nozzle and said chemical jetting nozzle being coupled to each other by a connecting arm member so that said vapour jetting nozzle and said chemical jetting nozzle confront each other to define a predetermined angle.

Thus there is provided a nozzle mechanism for an inhaler which is so designed as to protect a chemical from being diluted by water condensed from jetted steam.

Preferably, the inhaler cover includes a locking strip which can co-operate with any one of a first series of locking grooves on the sleeve to locate said sleeve in any one of a plurality of predetermined tilted positions relative to the cover, and said sleeve having a further locking groove co-operable with said locking strip to retain said sleeve in position relative to said cover when said sleeve is within the cover in said storage position.

In the accompanying drawings;

Figure 1 is a perspective view of an inhaler according to one example of the present invention;

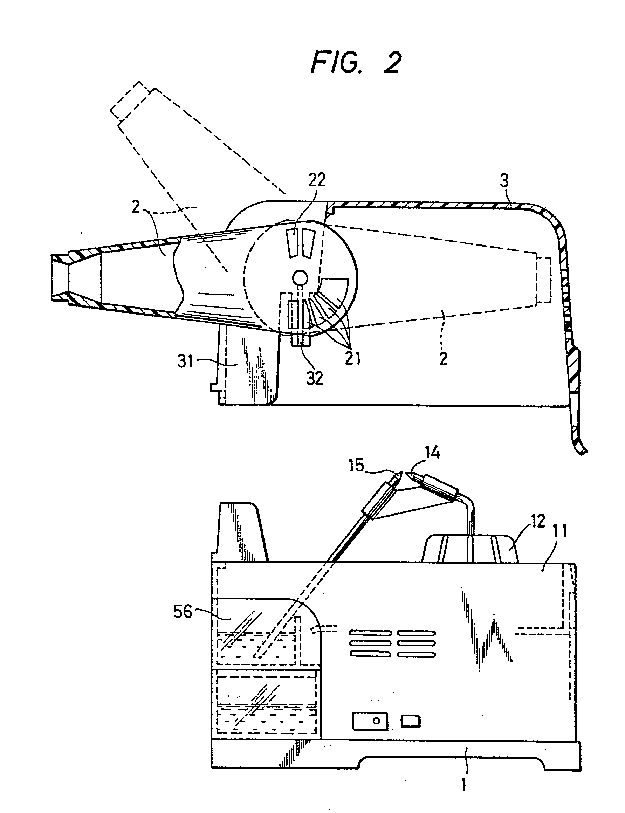

Figure 2 is an exploded view of the inhaler body and its cover, with part of the cover sectioned;

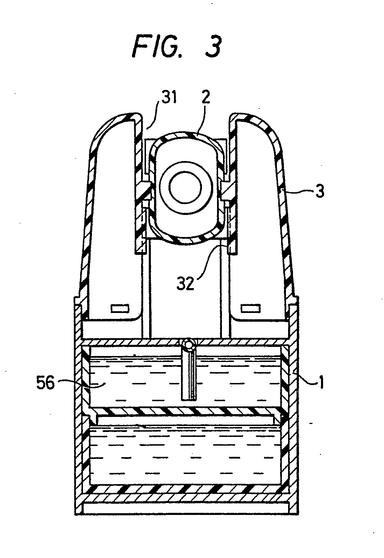

Figure 3 is a vertical sectional view of the inhaler shown in Figure 1;

Figure 4 is a perspective view of the inhaler from which the cover has been removed; and

Figure 5 is a side view of components of the inhaler shown in Figure 4.

Referring to the accompanying drawings, the inhaler comprises a vapour producing unit body 1 an inner construction of which will be described in detail later, and a cover 3 having a rotatable vapour feeding sleeve 2 arranged to feed a mixture of a chemical vapour and steam. It is preferable that the cover 3 be made of plastic. The vapour producing unit body 1 has a housing 11 on which a vapour producing unit 12 and a chemical container 56 are provided. The vapour producing unit 12 (an inner mechanism of which is well known in the art) is provided with a vapour jetting nozzle 14 and a chemical sucking nozzle 15. The cover 3 is detachably fitted in the upper opening of the vapour producing unit body 1. A nozzle path opening 31 is formed in the front portion of the cover 3. The sleeve 2 is pivotably mounted in the opening 31 in such a manner that it can be tilted at a desired angle to the cover 3. The sleeve 2 is substantially in the form of a tapered cylinder the diameter of which is gradually reduced towards the outer end. Locking grooves 21 and a locking groove 22 which is used when the nozzle is turned in an opposite direction are defined between ribs on each side wall of the sleeve 2, and are disposed radially around the axis of pivotal movement of the sleeve in such a manner that a selected one of the grooves on each side of the sleeve is engaged with by a respective locking strip 32 which protrudes from each side inner wall of the cover 3. The locking strip 32 protrudes from the inner wall of the cover 3 to the extent that the height of protrusion of the locking strip 32 will not prevent the rotation of the vapour feeding sleeve 2, for instance to 0.5 to 1 mm. Since each locking strip 32 is formed on a wall extending downwardly from the inner side wall of the cover 3 made of synthetic resin, the locking strips 32 firmly engaged with the selected grooves 21 or 22 formed in the side walls of the sleeve 2, to provide a detent action defining stable positions of the sleeve relative to the cover.

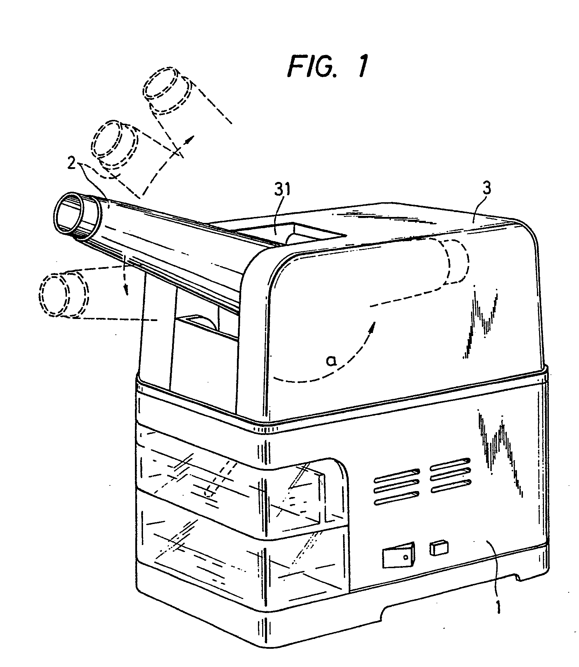

As is apparent from the above description the angle of inclination of the vapour feeding sleeve 2 can be changed. Therefore, the user can set the vapour feeding sleeve at a suitable angle to correspond to the position of a seated user so that the vapour is naturally applied to the throat of the user. Accordingly, even if the user uses the inhaler for a long time, he will not become fatigued because his posture is natural in using the inhaler. After the inhaler has been used, the sleeve 2 is turned in the direction of the arrow a in Figure 1 (after lifting the cover 3) until the locking grooves 22 are engaged by locking strips 32, so that the sleeve 2 is stably held in the cover 3. Therefore, the vapour feeding sleeve 2 is protected against contamination by being within the cover 3.

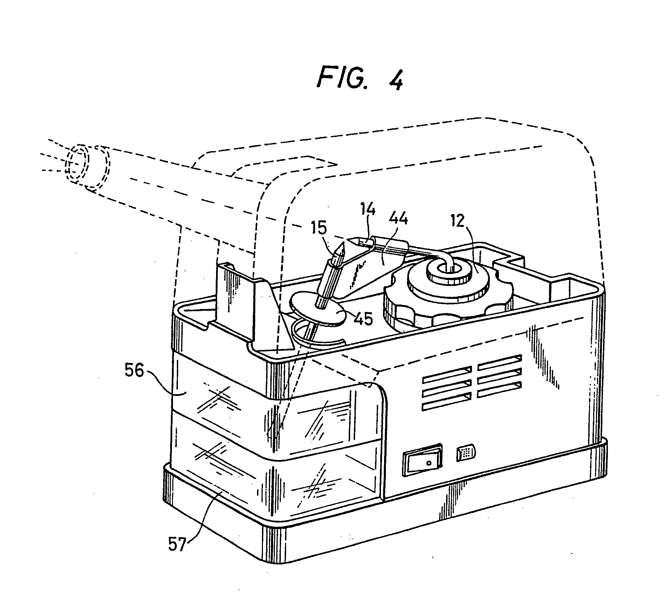

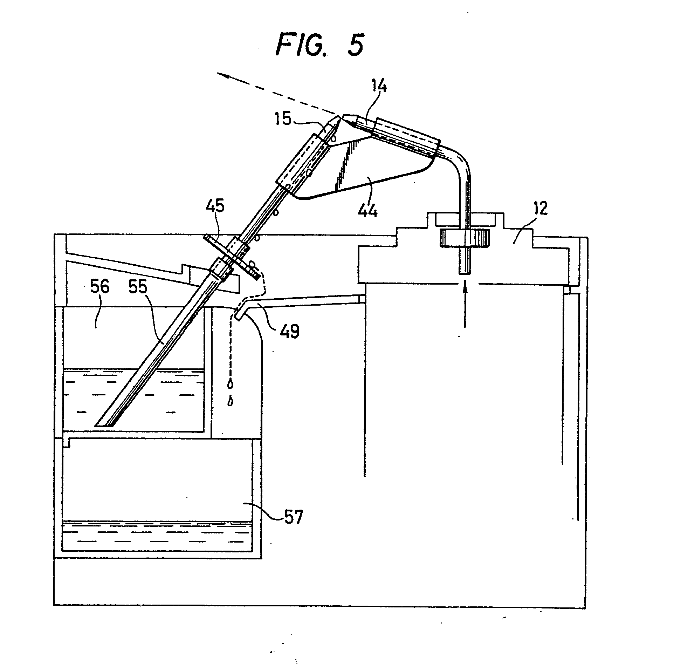

A preferred construction of an inner mechanism of the vapour producing unit body will now be described in detail with reference to Figures 4 and 5. In Figures 4 and 5, the water vapour jetting nozzle 14 communicates through its base with a water vapour producing unit 12 and the top end of the chemical sucking nozzle 15 is confronted with the top end of the nozzle 14 by means of an arm plate 44. A water by-passing flange 45 is secured to the base of the chemical sucking nozzle 15. The flange 45 extends radially from the base of the nozzle 15 to a suitable dimension. Further, in Figure 5, reference numeral 55 designates a tube of rubber or the like one end of which is coupled to the lower end portion of the chemical sucking nozzle 15, with the other end inserted into a chemical container 56.

As described above, the water by-passing flange 45 is provided integral with the base of the chemical sucking nozzle 15. Therefore, as shown in Figure 5, water condensed from the steam in the inhaler, which otherwise would flow down the sucking nozzle 15 into the chemical container 56, is directed into a condensed water receiving container 57 by the water by-passing flange 45 in association with a deflector 49. Thus, the chemical in the chemical container 56 is not diluted by the condensed water.

Only one preferred embodiment of the invention has been described. It is apparent that various modifications are possible within the scope of the accompanying claims.

## Claims

1. An inhaler comprising a body (1) including a vapour producing unit (12), a cover (3) covering said vapour producing unit (12), and a vapour feeding sleeve (2) for conducting vapour from the unit (12) to a user's mouth in use, said sleeve being supported by said cover (3) for tilting movement whereby the sleeve can be tilted at a desired angle for use, characterized in that said cover (3) is detachable from said body (1), and said sleeve can be rotated relative to said cover between a storage position wherein the sleeve is housed within the cover, and an operative position wherein the sleeve projects from the cover for use.

2. An inhaler as claimed in claim 1, characterized in that said cover (3) includes a locking strip (32) which can cooperate with any one of a first series of locking grooves (21) on the sleeve to locate said sleeve in any one of a plurality of predetermined tilted positions relative to the cover, and said sleeve having a further locking groove (22) co-operable with said locking strip (32) to retain said sleeve in position relative to said cover (3) when said sleeve (2) is within the cover in said storage position.

## Revendications

1. Inhalateur possédant un corps (1) qui comprend un générateur de vapeur (12), un couvercle (3) recouvrant le générateur de vapeur (12), ainsi qu'une tubulure d'éjection de vapeur (2) servant à amener de la vapeur du générateur (12) à la bouche d'un utilisateur, la tubulure étant supportée inclinable par le couvercle (3), de sorte que le tubulure peut être inclinée sous l'angle désiré pour l'utilisation, caractérisé en ce que le couvercle (3) est détachable du corps (1) et la tubulure peut être tournée, par rapport au convercle, entre une position de rangement où elle est logée dans le couvercle et une position d'utilisation où la tubulure fait saillie du couvercle.

2. Inhalateur selon la revendication 1, caractérisé en ce que le couvercle (3) présente une nervure de blocage (32) qui peut coopérer avec l'une quelconque d'une première série de rainures de blocage (21) sur la tubulure, en vue de l'immobilisation de la tubulure à l'une quelconque de plusieurs positions inclinées prédéterminées, par rapport au couvercle, la tubulure présentant une rainure de blocage (22) supplémentaire qui peut coopérer avec la nervure de blocage (32) pour immobiliser la tubulure par rapport au couvercle (3) lorsque la tubulure (2) occupe sa position de rangement à l'intérieur du couvercle.

## Patentansprüche

1. Inhalationsvorrichtung mit einem Körper (1), welcher eine Dampferzeugungseinheit (12), einen die Dampferzeugungseinheit (12) abdeckenden Deckel (3) und eine Dampflieferhülse (2) aufweist, um beim Gebrauch den Dampf von der Einheit (12) zum Mund eines Benutzer zu leiten, welche Hülse für eine Schwenkbewegung zom Deckel (3) abgestützt wird, wodurch die Hülse beim Gebrauch in die gewünschte Winkellage geschwenkt werden kann, dadurch gekennzeichnet, daß der Deckel (3) vom Körper (1) abnehmbar ist, und daß die Hülse relativ zum Deckel zwischen einer Aufbewahrungsstellung, in der die Hülse innerhalb des Deckels aufgenommon wird, und einer Betriebsstellung, in der die Hülse für den Gebrauch aus dem Deckel vorsteht, verschwenkt werden kann.

2. Inhalationsvorrichtung nach Anspruch 1, dadurch gekennzeichnet, daß der Deckel (3) einen Verriegelungsstreifen (32) umfaßt, welcher mit einer einer ersten Reihe von Verriegelungsnuten (21) an der Hülse zusammenwirken kann, um die Hülse in irgendeiner von einer Vielzahl von vorbestimmten Schwenklagen relativ zum Deckel zu lokalisieren, und daß die Hülse eine weitere Verriegelungsnut (22) aufweist, die mit dem Verriegelungsstreifen (32) zusammenwirkt, um die Hülse in der Lage relativ zum Deckel (3) zu halten, wenn sich die Hülse (2) innerhalb des Deckels in der Aufbewahrungsstellung befindet.

# FIG. 1

# FIG. 2

FIG. 3

FIG. 4

# FIG. 5